# EUROPÄISCHE PATENTSCHRIFT

(11) **EP 2 948 734 B1**
(45) Veröffentlichungstag und Bekanntmachung des Hinweises auf die Patenterteilung: **10.08.2022**
(21) Anmeldenummer: 13817926.2
(22) Anmeldetag: 20.12.2013
(51) Int. Cl.: G01B 15/02, G01N 22/04, G01N 33/36

(54) **MIKROWELLENRESONATOR FÜR EINE TEXTILMASCHINE**
MICROWAVE RESONATOR FOR A TEXTILE MACHINE
RÉSONATEUR À MICRO-ONDES POUR UNE MACHINE TEXTILE

(30) Priorität: 23.01.2013 DE 102013100644
(43) Veröffentlichungstag der Anmeldung: 02.12.2015
(73) Patentinhaber: Rieter Ingolstadt GmbH, 85055 Ingolstadt (DE)
(72) Erfinder: HERMANN, Tobias, 80798 München (DE); EIBERT, Thomas, 81925 München (DE); FAZ, Muhammad Usman, 81245 München (DE); SCHMIDT, Carsten, 81243 München (DE); UEDING, Michael, 85049 Ingolstadt (DE)
(74) Vertreter: Canzler & Bergmeier Patentanwälte Partnerschaft mbB
(86) Internationale Anmeldenummer: PCT/EP2013/077615
(87) Internationale Veröffentlichungsnummer: WO 2014/114418

(56) Entgegenhaltungen:
- WO-A2-03/085179
- DE-A1-102006 062 339
- US-A- 5 455 516

## Beschreibung

Die Erfindung betrifft einen Mikrowellenresonator für eine Messeinrichtung zur Messung der Dicke und/oder der Feuchtigkeit von kontinuierlich durch einen in einem Resonatorraum des Mikrowellenresonators angeordneten Messraum gefördertem strangförmigem Fasermaterial an einer Textilmaschine, insbesondere an einer Karde, an einer Strecke oder an einer Kämmmaschine.

Die Messung von Fasereigenschaften in der Textilindustrie ist eine unabdingbare Voraussetzung zur Produktion von hochwertigen Textilien. So ist beispielsweise die Messung von Fasermaterialdicken, insbesondere zum Zwecke der Ausregulierung von Ungleichmäßigkeiten von einem oder mehreren einer Spinnereivorbereitungsmaschine vorgelegten Faserbändern, unabdingbar. Gleichfalls ist zur Qualitätskontrolle des verstreckten Materials an einem Ausgang einer Spinnereivorbereitungsmaschine eine derartige Messung wünschenswert. Messwerte zur Faserbanddicke (es sind auch die Bezeichnungen Bandquerschnitt oder Bandmasse gebräuchlich) werden neben der genannten Qualitätskontrolle auch zum Abstellen der Maschine herangezogen, wenn vorgegebene Dickengrenzwerte überschritten werden und somit kein hochwertiges Produkt mehr erhalten wird.

Bisher werden überwiegend mechanisch abtastende Sensoren zur Ermittlung der Banddicke von dem bzw. den Faserbändern eingesetzt. Auch sind kapazitive Messorgane bekannt. Eine neue Methode zur Fasermaterialdickenmessung stellt hingegen die Verwendung von Mikrowellen dar. Hierbei werden von einem Mikrowellengenerator erzeugte Mikrowellen, deren Frequenzen bevorzugt von einem Rechner innerhalb gewisser Grenzen verändert werden, in einen Resonatorraum eines Mikrowellenresonators eingekoppelt, durch welchen auch das zu vermessende Fasermaterial kontinuierlich hindurchgeführt wird. Entsprechend der Faserart, der Materialdicken und Materialgeometrie sowie der Materialfeuchtigkeit tritt bei einer charakteristischen Mikrowellenfrequenz ein Resonanzsignal auf, welches nach Auskopplung von einem Rechner zur Ermittlung der Fasermaterialdicke und/oder der Fasermaterialfeuchtigkeit auswertbar ist. Eine derartige Methode für andere Anwendungszwecke ist beispielsweise in der EP 0 468 023 B1 beschrieben, deren Offenbarungsgehalt hiermit explizit eingeschlossen wird. Die Vorteile eines derartigen Messverfahrens mittels Mikrowellen liegen insbesondere darin, dass ein hochpräzises, berührungsloses Abtasten eines strangförmigen und schnelllaufenden Fasermaterials möglich ist. Mechanische Beeinträchtigungen des Fasermaterials sowie Messungenauigkeiten aufgrund der Trägheit von mechanischen Messelementen scheiden aus.

Ein Mikrowellenresonator mit einem elektrisch nicht leitenden Führungselement ist aus EP 2 390 391 A2 bekannt.

Es hat sich herausgestellt, dass diverse Probleme bezüglich des Zusammenspiels des Resonators und des hindurchgeführten Fasermaterials bestehen. So verändert sich beispielsweise bei der Entfernung des Fasermaterials aus dem Resonatorraum - z. B. bei auslaufender Maschine oder bei Entfernung eines Bandstaus - die Leerresonanzfrequenz mit der Zeit aufgrund eines Temperaturabfalls im Resonatorraum. Bei einem erneuten Einführen von Fasermaterial in den Resonator ist dieser daher verstimmt und muss neu eingestellt werden. Andere Probleme betreffen Schwankungen der Resonanzfrequenzen im Betrieb, die ebenfalls nicht von dem Fasermaterial herrühren. Es ist Aufgabe der vorliegenden Erfindung, einen Mikrowellenresonator zur Messung der Dicke bzw. Masse und/oder Feuchtigkeit von hindurchgefördertem strangförmigen Fasermaterial zu verbessern.

Diese Aufgabe wird bei einem Mikrowellenresonator der eingangs genannten Art dadurch gelöst, dass in dem Resonatorraum längs des Messraums wenigstens eine elektrisch nicht leitende Materialeinheit vorgesehen ist, welche einen eingangsseitigen Abschnitt mit einer in einer vorgesehenen Laufrichtung des Fasermaterials veränderlichen Querschnittsfläche und/oder einen ausgangsseitigen Abschnitt mit einer in der vorgesehenen Laufrichtung des Fasermaterials veränderlichen Querschnittsfläche aufweist, wobei die Querschnittsfläche des eingangsseitigen Abschnitts der Materialeinheit in der Laufrichtung des Fasermaterials gesehen zunimmt und/oder die Querschnittsfläche des ausgangsseitigen Abschnitts der Materialeinheit in der Laufrichtung des Fasermaterials gesehen abnimmt.

Unter dem Resonatorraum eines Mikrowellenresonators wird derjenige Raum verstanden, in dem sich bei Einkopplung von Mikrowellen ein elektro-magnetisches Feld ausbildet. Der Resonatorraum ist üblicherweise ein im Wesentlichen von einem leitenden Material umgebner Hohlraum, wobei in dem leitenden Material Aussparungen für einen Eintrittsbereich für das Fasermaterial sowie für einen Austrittsbereich für das Fasermaterial vorgesehen sind. Ebenso sind üblicherweise Aussparungen für eine Einkoppeleinrichtung zum Einkoppeln von Mikrowellen, für eine Auskoppeleinrichtung für Mikrowellen und/oder eine kombinierte Einkoppel/Auskoppeleinrichtung vorgesehen. Der Messraum ist derjenige Teil des Resonatorraums, durch den das zu vermessende Fasermaterial geführt ist. Er erstreckt sich im Allgemeinen von dem Eintrittsbereich bis zu dem Austrittsbereich, wobei der Messraum üblicherweise durch ein oder mehrere Begrenzungselemente konstruktiv vom übrigen Resonatorraum abgetrennt ist.

Weiterhin wird unter einer Materialeinheit ein zusammenhängendes ein- oder mehrstückiges Materialvolumen verstanden, wobei längs des Messraums bedeutet, dass sich die betreffende Materialeinheit entlang des Messraums in Laufrichtung wenigstens über einen Teil der Länge des Messraums erstreckt.

Die Querschnittsfläche der Materialeinheit ist eine Schnittfläche der Materialeinheit in einer zur Laufrichtung senkrechten Ebene. Dabei wird unter einem Abschnitt der Materialeinheit mit einer in der vorgesehenen Laufrichtung des Fasermaterials veränderlichen Querschnittsfläche ein solcher Abschnitt der Materialeinheit verstanden, bei dem sich die Querschnittsfläche in Laufrichtung über einen wesentlichen Teil der Erstreckung der Materialeinheit verändert.

Die Erfindung hat in einem ersten Schritt erkannt, dass Messungenauigkeiten bei gattungsgemäßen Mikrowellenresonatoren darauf zurückzuführen sind, dass sich ein schnelllaufendes Fasermaterial im Messraum quer zur Laufrichtung unregelmäßig hin- und herbewegt. Versuche, in einem zweiten Schritt, den Messraum quer zur Laufrichtung zu verkleinern, haben allerdings ergeben, dass hierdurch die Reibung zwischen Fasermaterial und dem oder den Begrenzungselementen stark ansteigt und damit zu einem erhöhten Verschleiß des oder der Begrenzungselemente und auch zur Schädigung des Fasermaterials führt. In einem dritten Schritt hat die Erfindung erkannt, dass die Messungenauigkeiten bei einem sich quer zur Laufrichtung bewegenden Fasermaterial darauf zurückzuführen sind, dass der Betrag des elektrischen Felds im Messraum quer zur Laufrichtung des Fasermaterials variiert.

Dabei hat die Erfindung in einem vierten Schritt erkannt, dass eine sich längs des Messraums erstreckende Materialeinheit mit einem eingangsseitigen Abschnitt, dessen Querschnittsfläche in der Laufrichtung des Fasermaterials gesehen zunimmt, und/oder mit einem ausgangsseitigen Abschnitt, dessen Querschnittsfläche in der Laufrichtung des Fasermaterials gesehen abnimmt, dazu geeignet ist, den Betrag des elektrischen Felds im Messraum in Querrichtung zu vergleichmäßigen. Hierdurch ist es möglich, die in der Praxis zu erreichende Messgenauigkeit, insbesondere bei höheren Geschwindigkeiten des Fasermaterials, zu verbessern.

Der erfindungsgemäße Mikrowellenresonator eignet sich prinzipiell für die Vermessung aller strangförmigen Fasermaterialien, insbesondere jedoch für Faserbänder, Vorgarne und Garne, wobei auch vorgesehen sein kann, mehrere strangförmige Fasermaterialien gleichzeitig und gemeinsam zu vermessen, um so beispielsweise die Gesamtmasse von mehreren Faserbändern zu bestimmen.

Gemäß einer vorteilhaften Weiterbildung der Erfindung ist vorgesehen, dass die Querschnittsfläche des eingangsseitigen Abschnitts der Materialeinheit kontinuierlich zunimmt und/oder dass die Querschnittsfläche des ausgangsseitigen Abschnitts der Materialeinheit kontinuierlich abnimmt. Unter kontinuierlich wird hierbei verstanden, dass sich die Querschnittsfläche stetig, also ohne Sprünge verändert. Prinzipiell sind auch Ausführungsbeispiele denkbar, bei denen die Querschnittsfläche der Materialeinheit stufenartig zu- bzw. abnimmt, allerdings hat es sich gezeigt, dass bei einem kontinuierlichen Querschnittsverlauf ein wesentlich größerer Effekt hinsichtlich der Homogenisierung des elektrischen Felds quer zur Laufrichtung erzielt werden kann, so dass die Messgenauigkeit weiter verbessert wird.

Nach einer vorteilhaften Weiterbildung der Erfindung grenzen der eingangsseitige Abschnitt der Materialeinheit und der ausgangsseitige Abschnitt der Materialeinheit aneinander an. D. h. es ist zwischen dem eingansseitigen Abschnitt und dem ausgangsseitigen Abschnitt kein Bereich mit einer wesentlichen Erstreckung in Laufrichtung vorgesehen, in dem die Materialeinheit einen gleichbleibenden Querschnitt aufweist. Auf diese Weise kann das elektrische Feld besonders günstig beeinflusst werden.

Gemäß einer vorteilhaften Weiterbildung der Erfindung ist vorgesehen, dass sich der eingangsseitige Abschnitt von einem Eintrittsbereich des Fasermaterials in den Resonatorraum bis zu einem mittleren Bereich des Resonatorraums erstreckt und/oder dass sich der ausgangsseitige Abschnitt von dem mittleren Bereich des Resonatorraums bis zu einem Austrittsbereich des Fasermaterials aus dem Resonatorraum erstreckt. Auch auf diese Weise kann das elektrische Feld besonders günstig beeinflusst werden.

Nach einer vorteilhaften Weiterbildung der Erfindung sind der eingangsseitige Abschnitt und der ausgangsseitige Abschnitt spiegelsymmetrisch zueinander ausgebildet. Spiegelsymmetrie, auch Ebenensymmetrie genannt, ist eine Verallgemeinerung der Achsensymmetrie in den dreidimensionalen Raum. Allgemein ist ein dreidimensionaler Körper dann spiegelsymmetrisch, falls es eine solche Ebene gibt, an der eine Spiegelung des Körpers diesen auf sich selber abbildet. Im konkreten Fall kann die Spiegelebene insbesondere senkrecht zur Laufrichtung des Fasermaterials ausgerichtet sein. Hierdurch kann die Homogenisierung des elektrischen Felds weiter verbessert werden.

Gemäß einer vorteilhaften Weiterbildung der Erfindung ist vorgesehen, dass die Materialeinheit an der dem Messraum abgewandten Seite eines den Messraum begrenzenden Begrenzungselements zur Führung des Fasermaterials und/oder zur Abdeckung eines Hohlraumbereichs des Resonatorraums angeordnet ist. Derartige Begrenzungselemente dienen der passiven Führung des Fasermaterials und/oder der Abdeckung eines nicht zum Messraum gehörenden Hohlraums des Resonatorraums, um dort insbesondere eine Ablagerung von messwertverfälschenden Partikeln, wie Staub, zu verhindern. Wird nun die Materialeinheit an der dem Messraum abgewandten Seite des den Messraum begrenzenden Begrenzungselements, also insbesondere im abgedeckten Hohlraum angeordnet, so ist eine mechanische Einwirkung des Fasermaterials auf die Materialeinheit ausgeschlossen, so dass dort keinerlei Verschleiß auftritt.

Nach einer vorteilhaften Weiterbildung der Erfindung ist die Materialeinheit als ein den Messraum begrenzendes Begrenzungselement zur Führung des Fasermaterials ausgebildet. Erfindungsgemäß ist die Materialeinheit ferner zur Abdeckung eines Hohlraumbereichs des Resonatorraums ausgebildet. Durch die mögliche Zusammenfassung der feldhomogenisierenden Materialeinheit und einem Begrenzungselement kann der konstruktive Aufwand für den Aufbau des Mikrowellenresonators verringert werden und gleichzeitig die gewünschte Homogenisierung des Felds erreicht werden.

Gemäß einer vorteilhaften Weiterbildung der Erfindung ist vorgesehen, dass die Materialeinheit aus einer Keramik, insbesondere aus Aluminiumoxid, aus einem Glas, insbesondere aus Quarzglas, aus einem Verbundwerkstoff mit eingebetteter Keramik, insbesondere aus TMM^{®}, oder aus einem Kunststoff, insbesondere aus Polyethylen, hergestellt ist. Die genannten Materialien zeichnen sich durch eine in einem mittleren Bereich liegende Dielektrizitätskonstante aus, so dass das elektrische Feld mit einer vergleichsweise geringen Materialmenge hinreichend beeinflusst werden kann. Allerdings ergeben sich noch hinreichende Materialstärken, welche die mechanische Stabilität der Materialeinheiten gewährleisten. Darüber hinaus weisen die Materialien einen geringen Verlustfaktor auf, so dass die Güte des Mikrowellenresonators nur wenig beeinflusst wird. Zudem sind die elektrischen und mechanischen Eigenschaften, wie beispielsweise die Dielektrizitätskonstante oder die Länge, weitgehend temperaturunabhängig, so dass im Betrieb auftretende Temperaturschwankungen allenfalls zu untergeordneten Messungenauigkeiten führen. Keramik, Glas und Verbundwerkstoffe mit eingebetteter Keramik sind zudem sehr abriebfest, so dass sie insbesondere für Materialeinheiten geeignet sind, welche als Begrenzungselement ausgebildet sind.

Nach einer vorteilhaften Weiterbildung der Erfindung ist der Resonatorraum zylindrisch, insbesondere ellipsenzylindrisch oder kreiszylindrisch geformt, wobei die Laufrichtung des Fasermaterials parallel zu einer Zylinderachse des Resonatorraums verläuft und wobei bevorzugt die Materialeinheit rotationssymmetrisch ausgebildet und koaxial zur Zylinderachse angeordnet ist. Zylindrische Resonatorräume, insbesondere ellipsenzylindrische oder kreiszylindrische Resonatorräume, bei denen die Laufrichtung des Fasermaterials parallel zu einer Zylinderachse des Resonatorraums verläuft, eignen sich insbesondere zur Vermessung von einem einzigen strangförmigen Fasermaterial mit einem runden oder ovalen Querschnitt. In solchen Fällen kann das elektrische Feld im Messraum durch eine rotationssymmetrische Materialeinheit, also durch eine relativ einfach geformte Materialeinheit, weitestgehend homogenisiert werden.

Gemäß einer vorteilhaften Weiterbildung der Erfindung ist vorgesehen, dass die Materialeinheit eine zylindrische Öffnung, bevorzugt eine ellipsen- oder kreiszylindrische Öffnung aufweist, die parallel, bevorzugt koaxial, zur Zylinderachse des Resonatorraums verläuft, wobei sich die Außenseite des eingangsseitigen Abschnitts in Laufrichtung konisch erweitert und/oder wobei sich die Außenseite des ausgangsseitigen Abschnitts in Laufrichtung konisch verjüngt. Mit dieser einfach herzustellenden Geometrie der Materialeinheit kann insbesondere bei einem zylindrischen Resonatorraum, bei dem die Laufrichtung des Fasermaterials parallel zu einer Zylinderachse des Resonatorraums verläuft, das elektrische Feld im Messraum in besonders einfacher Weise homogenisiert werden.

Nach einer vorteilhaften Weiterbildung der Erfindung ist vorgesehen, dass die Materialeinheit eine zylindrische Öffnung, bevorzugt eine ellipsen- oder kreiszylindrische Öffnung, aufweist, die parallel, bevorzugt koaxial, zur Zylinderachse des Resonatorraums verläuft, wobei sich die Außenseite des eingangsseitigen Abschnitts in Laufrichtung konvex gewölbt erweitert und/oder wobei sich die Außenseite des ausgangsseitigen Abschnitts in Laufrichtung konvex gewölbt verjüngt. Mit dieser etwas aufwendiger herzustellenden Geometrie der Materialeinheit kann, insbesondere bei einem zylindrischen Resonatorraum, bei dem die Laufrichtung des Fasermaterials parallel zu einer Zylinderachse des Resonatorraums verläuft, das elektrische Feld im Messraum noch wirkungsvoller homogenisiert werden.

Nach einer vorteilhaften Weiterbildung der Erfindung ist vorgesehen, dass der Mikrowellenresonator zum Betrieb in einem TM₀ₙ₀-Resonanzmodus ausgebildet ist, wobei n eine natürliche Zahl, bevorzugt größer als 1 und besonders bevorzugt größer als 2 ist. Unter einem TM-Resonanzmodus (Transversal Magnetischer Resonanzmodus) wird bei einem zylindrischen Resonatorraum allgemein ein Resonanzmodus verstanden, bei dem kein magnetisches Feld in Richtung der Zylinderachse, hier also in Laufrichtung, auftritt. Dies ist insofern günstig, als bei der Vermessung von Fasermaterialien hauptsächlich die Wechselwirkung zwischen dem jeweiligen Fasermaterial und dem elektrischen Feld der Mikrowellen auswertbar ist, wobei diese Wechselwirkung bei einem zylindrischen Resonatorraum, bei dem die Zylinderachse parallel zur Laufrichtung ist, in einem TM-Resonanzmodus besonders ausgeprägt ist. Der Index n gibt dabei an, wie viele Halbwellen das elektrische Feld in einer Richtung quer zur Zylinderachse ausbildet. Technisch bedingt beträgt n mindestens 1, da sich im entsprechenden Resonanzfall mindestens eine derartige Halbwelle ausbildet. Dabei hat sich jedoch gezeigt, dass sich das elektrische Feld im Messraum günstiger, beispielsweise homogener, ausbildet, wenn die Anzahl der Halbwellen erhöht wird, n also größer gewählt wird. Der Grund hierfür liegt darin, dass sich bei gegebener Geometrie des Mikrowellenresonators eine höhere Resonanzfrequenz ergibt.

Nach einer vorteilhaften Weiterbildung der Erfindung ist vorgesehen, dass der Resonatorraum zylindrisch, insbesondere ellipsenzylindrisch oder kreiszylindrisch geformt ist, wobei die Laufrichtung des Fasermaterials quer zu einer Zylinderachse des Resonatorraums verläuft und wobei der Messraum im Querschnitt als länglicher Schlitz ausgebildet ist, wobei eine erste Materialeinheit längs einer ersten Breitseite des Schlitzes und eine zweite Materialeinheit längs einer gegenüberliegenden zweiten Breitseite des Schlitzes angeordnet ist, wobei bevorzugt die erste Materialeinheit und die zweite Materialeinheit bezüglich einer durch die Zylinderachse und parallel zur Laufrichtung verlaufenden Symmetrieebene spiegelsymmetrisch zueinander angeordnet sind. Zylindrische Resonatorräume, insbesondere ellipsenzylindrische oder kreiszylindrische Resonatorräume, bei denen die Laufrichtung des Fasermaterials quer zu einer Zylinderachse des Resonatorraums verläuft und bei denen der Messraum als länglicher Schlitz ausgebildet ist, eignen sich insbesondere zur gemeinsamen Vermessung von mehreren nebeneinanderlaufenden strangförmigen Fasermaterialen oder von einem strangförmigen Fasermaterial mit länglichem Querschnitt. In solchen Fällen kann das elektrische Feld im Messraum durch eine erste Materialeinheit, welche längs einer ersten Breitseite des Schlitzes angeordnet ist, und durch eine zweite Materialeinheit, welche längs einer gegenüberliegenden zweiten Breitseite des Schlitzes angeordnet ist, also durch eine relativ einfache Anordnung von zwei Materialeinheiten, positiv beeinflusst, beispielsweise weitestgehend homogenisiert, werden. Dabei kann eine besonders wirksame Beeinflussung des elektrischen Felds erreicht werden, wenn die erste Materialeinheit und die zweite Materialeinheit bezüglich einer durch die Zylinderachse und parallel zur Laufrichtung verlaufenden Symmetrieebene spiegelsymmetrisch zueinander angeordnet sind.

Nach einer vorteilhaften Weiterbildung der Erfindung ist vorgesehen, dass die erste Materialeinheit und/oder die zweite Materialeinheit eine parallel zur Zylinderachse verlaufende im Wesentlichen ebene Innenseite aufweist, wobei sich die Außenseite des jeweiligen eingangsseitigen Abschnitts in Laufrichtung keilförmig erweitert und/oder wobei sich die Außenseite des jeweiligen ausgangsseitigen Abschnitts in Laufrichtung keilförmig verjüngt. Mit dieser einfach herzustellenden Geometrie der Materialeinheiten kann, insbesondere bei einem zylindrischen Resonatorraum, bei dem die Laufrichtung des Fasermaterials quer zu einer Zylinderachse des Resonatorraums verläuft, das elektrische Feld im Messraum in besonders einfacher Weise beeinflusst, beispielsweise homogenisiert, werden.

Nach einer vorteilhaften Weiterbildung der Erfindung ist vorgesehen, dass die erste Materialeinheit und/oder die zweite Materialeinheit eine parallel zur Zylinderachse verlaufende im Wesentlichen ebene Innenseite aufweist, wobei sich die Außenseite des jeweiligen eingangsseitigen Abschnitts in Laufrichtung konvex gewölbt erweitert und/oder wobei sich die Außenseite des jeweiligen ausgangsseitigen Abschnitts in Laufrichtung konvex gewölbt verjüngt. Mit dieser etwas aufwendiger herzustellenden Geometrie der Materialeinheiten kann, insbesondere bei einem zylindrischen Resonatorraum, bei dem die Laufrichtung des Fasermaterials quer zu einer Zylinderachse des Resonatorraums verläuft, das elektrische Feld im Messraum noch wirkungsvoller beeinflusst, beispielsweise homogenisiert, werden.

Nach einer vorteilhaften Weiterbildung der Erfindung ist vorgesehen, dass der Mikrowellenresonator zum Betrieb in einem TE₀ₙₘ-Resonanzmodus ausgebildet ist, wobei n größer als 0, bevorzugt größer als 1 und besonders bevorzugt größer als 2 ist. Unter einem TE-Resonanzmodus (Transversal Elektrischer Resonanzmodus) wird bei einem zylindrischen Resonatorraum allgemein ein Resonanzmodus verstanden, bei dem kein elektrisches Feld in Richtung der Zylinderachse auftritt. Dies ist insofern günstig, als bei der Vermessung von Fasermaterialien hauptsächlich die Wechselwirkung zwischen dem jeweiligen Fasermaterial und dem elektrischen Feld der Mikrowellen auswertbar ist, wobei diese Wechselwirkung bei einem zylindrischen Resonatorraum, bei dem die Zylinderachse quer zur Laufrichtung ist, in einem TE-Resonanzmodus besonders ausgeprägt ist. Der Index n gibt dabei an, wie viele Halbwellen das elektromagnetische Feld in einer Richtung quer zur Zylinderachse ausbildet. Technisch bedingt beträgt n mindestens 1, da sich im entsprechenden Resonanzfall mindesten eine derartige Halbwelle ausbildet. Dabei hat sich jedoch gezeigt, dass sich das elektrische Feld im Messraum homogener ausbildet, wenn die Anzahl der Halbwellen erhöht wird, n also größer gewählt wird. Wird n jedoch größer gewählt, so muss bei gegebenem Resonatorraum die Frequenz vergrößert werden. Dabei ergibt sich ein guter Kompromiss aus Größe und Homogenität, wenn n zwischen 2 und 4 gewählt wird. Der Index m ist die Anzahl der Halbwellen entlang der Zylinderachse und ist typischerweise gleich 1.In einem weiteren Aspekt betrifft die Erfindung eine Textilmaschine, insbesondere Karde, Strecke oder Kämmmaschine, welche mindestens einen erfindungsgemäßen Mikrowellenresonator aufweist. Es ergeben sich die oben beschriebenen Vorteile.

Darüber hinaus betrifft die Erfindung eine Materialeinheit für einen erfindungsgemäßen Mikrowellenresonator, wodurch sich die oben beschriebenen Vorteile ergeben.

Die in den abhängigen Ansprüchen wiedergegebenen und/oder vorstehend erläuterten vorteilhaften Weiterbildungen der Erfindung können einzeln oder in beliebiger Kombination vorgesehen sein.

Die Erfindung und ihre Weiterbildungen sowie deren Vorteile sind nachfolgend anhand von Figuren näher erläutert. Es zeigen:
- **Figur 1**: ein erstes Ausführungsbeispiel eines erfindungsgemäßen Mikrowellenresonators in einer schematischen, geschnittenen Seitenansicht,
- **Figur 2**: den Mikrowellenresonator der Figur 1 in einer Aufsicht bei abgenommenem Deckel,
- **Figur 3**: ein zweites Ausführungsbeispiel eines erfindungsgemäßen Mikrowellenresonators in einer schematischen, geschnittenen Seitenansicht,
- **Figur 4**: den Mikrowellenresonator der Figur 3 in einer Aufsicht bei abgenommenem Deckel,
- **Figur 5**: ein drittes Ausführungsbeispiel eines erfindungsgemäßen Mikrowellenresonators in einer schematischen, geschnittenen Seitenansicht,
- **Figur 6**: den Mikrowellenresonator der Figur 5 in einer Aufsicht bei abgenommener oberer Halbschale,
- **Figur 7**: ein drittes Ausführungsbeispiel eines erfindungsgemäßen Mikrowellenresonators in einer schematischen, geschnittenen Seitenansicht, und
- **Figur 8**: den Mikrowellenresonator der Figur 7 in einer Aufsicht bei abgenommener oberer Halbschale.

In den folgenden Figuren sind einander entsprechende Teile mit denselben Bezugszeichen versehen. Dabei sind nur diejenigen Bestandteile eines Mikrowellenresonators mit Bezugszeichen versehen und erläutert, welche für das Verständnis der Erfindung erforderlich sind. Es versteht sich von selbst, dass der erfindungsgemäße Mikrowellenresonator weitere Teile und Baugruppen umfassen kann.

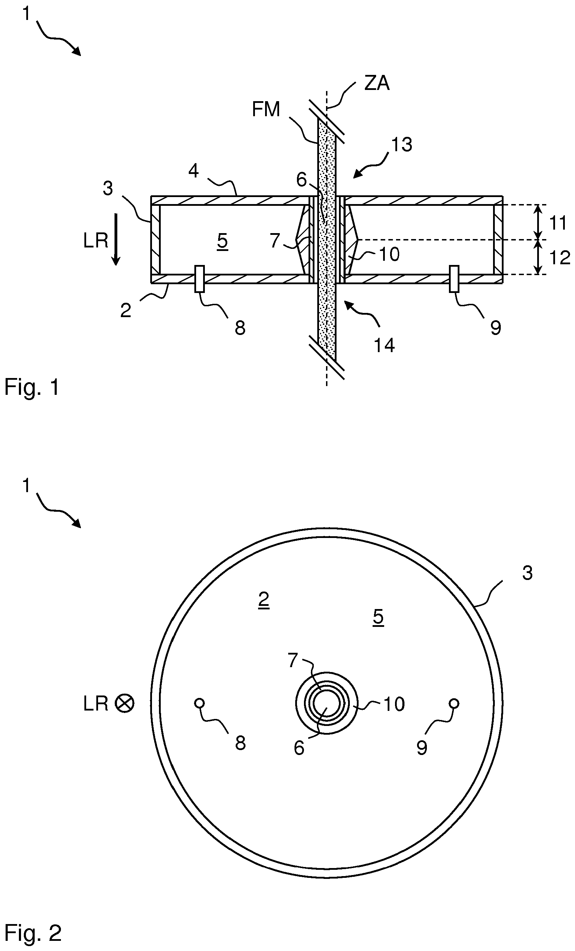

Figur 1 zeigt ein erstes Ausführungsbeispiel eines erfindungsgemäßen Mikrowellenresonators 1 in einer schematischen, geschnittenen Seitenansicht, welcher in Figur 2 in einer Aufsicht dargestellt ist. Der Mikrowellenresonator 1 weist ein Gehäuse 2, 3, 4 auf, welches durch einen runden Boden 2, eine kreiszylindermantelförmige Seitenwand 3 und einen runden Deckel 4 gebildet ist. Das Gehäuse 2, 3, 4 umfasst ein leitendes Material, so dass ein leitend umschlossener und im Wesentlichen geschlossener Resonatorraum 5 gebildet ist. Ein Teil des Resonatorraums 5 ist als Messraum 6 vorgesehen, der durch ein Begrenzungselement 7 vom übrigen Resonatorraum 5 abgetrennt ist. Der Messraum 6 ist vorgesehen, um darin ein kontinuierlich in einer Laufrichtung LR hindurchgeführtes strangförmiges Fasermaterial FM hinsichtlich seiner Masse bzw. Dicke und/oder Feuchtigkeit zu vermessen. Das Begrenzungselement 7 ist dabei zur Führung des Fasermaterials FM und/oder zur Abdeckung eines Hohlraumbereichs des Resonatorraums 5 ausgebildet.

Um das Fasermaterial FM zu vermessen, werden mittels einer Einkoppelanordnung 8 elektromagnetische Wellen mit variierender Frequenz in den Resonatorraum eingekoppelt. Ein Teil der Mikrowellen wird zeitgleich mit einer Auskoppelanordnung 9 aus dem Resonatorraum 5 ausgekoppelt, um daraus eine Resonanzkurve zu ermitteln. Dabei kann aus der Resonanzfrequenz der Resonanzkurve und der Halbwertsbreite der Resonanzkurve auf die Masse und/oder die Feuchte des Fasermaterials FM geschlossen werden, da sich die beiden Parameter Resonanzfrequenz und Halbwertsbreite aufgrund der Wechselwirkung des Fasermaterials FM und insbesondere der elektrischen Komponente der Mikrowellen spezifisch einstellen.

Die Erfindung hat in einem ersten Schritt erkannt, dass Messungenauigkeiten bei bekannten Mikrowellenresonatoren 1 darauf zurückzuführen sind, dass sich ein schnelllaufendes Fasermaterial FM im Messraum 6 quer zur Laufrichtung LR unregelmäßig hin- und herbewegt. Versuche, in einem zweiten Schritt, den Messraum 6 quer zur Laufrichtung LR zu verkleinern, haben allerdings ergeben, dass hierdurch die Reibung zwischen Fasermaterial FM und dem oder den Begrenzungselementen 7 stark ansteigt und damit zu einem erhöhten Verschleiß des oder der Begrenzungselemente 7 und auch zur Schädigung des Fasermaterials FM führt. In einem dritten Schritt hat die Erfindung erkannt, dass die Messungenauigkeiten bei einem sich quer zur Laufrichtung bewegenden Fasermaterial FB darauf zurückzuführen sind, dass der Betrag des elektrischen Felds im Messraum 6 quer zur Laufrichtung LR des Fasermaterials FM variiert.

Dabei hat die Erfindung in einem vierten Schritt erkannt, dass eine sich längs des Messraums 6 erstreckende Materialeinheit 10 mit einem eingangsseitigen Abschnitt 11, dessen Querschnittsfläche in der Laufrichtung LR des Fasermaterials FM gesehen zunimmt, und/oder mit einem ausgangsseitigen Abschnitt 12, dessen Querschnittsfläche in der Laufrichtung LR des Fasermaterials FM gesehen abnimmt, dazu geeignet ist, den Betrag des elektrischen Felds im Messraum 6 in Querrichtung zu vergleichmäßigen. Hierdurch ist es möglich, die in der Praxis zu erreichende Messgenauigkeit, insbesondere bei höheren Geschwindigkeiten des Fasermaterials FM, zu verbessern. Dabei wird unter einer Materialeinheit 10 ein zusammenhängendes ein- oder mehrstückiges Materialvolumen verstanden, wobei längs des Messraums 6 bedeutet, dass sich die betreffende Materialeinheit 10 entlang des Messraums 6 in Laufrichtung wenigstens über einen Teil der Länge des Messraums 6 erstreckt.

Vorteilhafterweise ist vorgesehen, dass die Querschnittsfläche des eingangsseitigen Abschnitts 11 der Materialeinheit 10 kontinuierlich zunimmt und/oder dass die Querschnittsfläche des ausgangsseitigen Abschnitts 12 der Materialeinheit 10 kontinuierlich abnimmt. Prinzipiell sind auch Ausführungsbeispiele denkbar, bei denen die Querschnittsfläche der Materialeinheit 10 stufenartig zu- bzw. abnimmt, allerdings hat es sich gezeigt, dass bei einem kontinuierlichen Querschnittsverlauf ein wesentlich größerer Effekt hinsichtlich der Homogenisierung des elektrischen Felds quer zur Laufrichtung LR erzielt werden kann, so dass die Messgenauigkeit weiter verbessert wird. Vorteilhafterweise grenzen der eingangsseitige Abschnitt 11 der Materialeinheit 10 und der ausgangsseitige Abschnitt 12 der Materialeinheit 10 aneinander an. D. h. es ist zwischen dem eingansseitigen Abschnitt 11 und dem ausgangsseitigen Abschnitt 12 kein Bereich mit einer wesentlichen Erstreckung in Laufrichtung LR vorgesehen, in dem die Materialeinheit 10 einen gleichbleibenden Querschnitt aufweist. Auf diese Weise kann das elektrische Feld besonders günstig beeinflusst werden.

Zweckmäßigerweise ist vorgesehen, dass sich der eingangsseitige Abschnitt 11 von einem Eintrittsbereich 13 des Fasermaterials FM in den Resonatorraum 5 bis zu einem mittleren Bereich des Resonatorraums 5 erstreckt und/oder dass sich der ausgangsseitige Abschnitt 12 von dem mittleren Bereich des Resonatorraums 5 bis zu einem Austrittsbereich 14 des Fasermaterials FM aus dem Resonatorraum 5 erstreckt. Auch auf diese Weise kann das elektrische Feld besonders günstig beeinflusst werden.

Vorteilhafterweise sind der eingangsseitige Abschnitt 11 und der ausgangsseitige Abschnitt 12 spiegelsymmetrisch zueinander ausgebildet. Spiegelsymmetrie, auch Ebenensymmetrie genannt, ist eine Verallgemeinerung der Achsensymmetrie in den dreidimensionalen Raum. Allgemein ist ein dreidimensionaler Körper dann spiegelsymmetrisch, falls es eine solche Ebene gibt, an der eine Spiegelung des Körpers diesen auf sich selber abbildet. Im konkreten Fall kann die Spiegelebene insbesondere senkrecht zur Laufrichtung des Fasermaterials ausgerichtet Hierdurch kann die Homogenisierung des elektrischen Felds weiter verbessert werden.

Im ersten Ausführungsbeispiel ist vorgesehen, dass die Materialeinheit 10 an der dem Messraum 6 abgewandten Seite des den Messraum 6 begrenzenden Begrenzungselements 7 zur Führung des Fasermaterials FM und/oder zur Abdeckung eines Hohlraumbereichs des Resonatorraums 5 angeordnet ist. Wird nun die Materialeinheit 10 an der dem Messraum 6 abgewandten Seite des den Messraum 6 begrenzenden Begrenzungselements 7, also insbesondere im abgedeckten Hohlraum angeordnet, so ist eine mechanische Einwirkung des Fasermaterials FM auf die Materialeinheit 10 ausgeschlossen, so dass dort keinerlei Verschleiß auftritt.

Bevorzugt ist vorgesehen, dass die Materialeinheit 10 aus einer Keramik, insbesondere aus Aluminiumoxid, aus einem Glas, insbesondere aus Quarzglas, aus einem Verbundwerkstoff mit eingebetteter Keramik, insbesondere aus TMM^{®}, oder aus einem Kunststoff, insbesondere aus Polyethylen, hergestellt ist. Die genannten Materialien zeichnen sich durch eine in einem mittleren Bereich liegende Dielektrizitätskonstante aus, so dass das elektrische Feld mit einer vergleichsweise geringen Materialmenge hinreichend beeinflusst werden kann. Allerdings ergeben sich noch hinreichende Materialstärken, welche die mechanische Stabilität der Materialeinheiten gewährleisten. Darüber hinaus weisen die Materialien einen geringen Verlustfaktor auf, so dass die Güte des Mikrowellenresonators nur wenig beeinflusst wird. Zudem sind die elektrischen und mechanischen Eigenschaften, wie beispielsweise die Dielektrizitätskonstante oder die Länge, weitgehend temperaturunabhängig, so dass im Betrieb auftretende Temperaturschwankungen allenfalls zu untergeordneten Messungenauigkeiten führen. Besonders geeignet für die Materialeinheit 10 des ersten Ausführungsbeispiels sind jedoch kostengünstige und einfach zu formende Kunststoffe, da es hier auf eine Abriebfestigkeit nicht ankommt.

Zweckmäßigerweise ist der Resonatorraum 5 zylindrisch, insbesondere ellipsenzylindrisch oder kreiszylindrisch geformt, wobei die Laufrichtung LR des Fasermaterials FM parallel zu einer Zylinderachse ZA des Resonatorraums 5 verläuft und wobei bevorzugt die Materialeinheit 10 rotationssymmetrisch ausgebildet und koaxial zur Zylinderachse ZA angeordnet ist. Zylindrische Resonatorräume 5, insbesondere ellipsenzylindrische oder kreiszylindrische Resonatorräume 5, bei denen die Laufrichtung LR des Fasermaterials FM parallel zu einer Zylinderachse ZA des Resonatorraums 5 verläuft, eignen sich insbesondere zur Vermessung von einem einzigen strangförmigen Fasermaterial FM mit einem runden oder ovalen Querschnitt. In solchen Fällen kann das elektrische Feld im Messraum 6 durch eine rotationssymmetrische Materialeinheit 10, also durch eine relativ einfach geformte Materialeinheit 10, weitestgehend homogenisiert werden.

Zweckmäßigerweise ist vorgesehen, dass die Materialeinheit 10 eine zylindrische Öffnung, bevorzugt eine ellipsen- oder kreiszylindrische Öffnung aufweist, die parallel, bevorzugt koaxial, zur Zylinderachse ZA des Resonatorraums 5 verläuft, wobei sich die Außenseite des eingangsseitigen Abschnitts 11 in Laufrichtung LR konisch erweitert und/oder wobei sich die Außenseite des ausgangsseitigen Abschnitts 12 in Laufrichtung LR konisch verjüngt. Mit dieser einfach herzustellenden Geometrie der Materialeinheit 10 kann, insbesondere bei einem zylindrischen Resonatorraum 5 , bei dem die Laufrichtung LR des Fasermaterials FM parallel zu einer Zylinderachse ZA des Resonatorraums 5 verläuft, das elektrische Feld im Messraum 6 in besonders einfacher Weise homogenisiert werden.

Vorteilhafterweise ist vorgesehen, dass der Mikrowellenresonator 1 zum Betrieb in einem TM₀ₙ₀-Resonanzmodus ausgebildet ist, wobei n eine natürliche Zahl, bevorzugt größer als 1 und besonders bevorzugt größer als 2, ist. Unter einem TM-Resonanzmodus (Transversal Magnetischer Resonanzmodus) wird bei einem zylindrischen Resonatorraum 5 allgemein ein Resonanzmodus verstanden, bei dem kein magnetisches Feld in Richtung der Zylinderachse ZA, hier also in Laufrichtung LR, auftritt. Dies ist insofern günstig, als bei der Vermessung von Fasermaterialien FM hauptsächlich die Wechselwirkung zwischen dem jeweiligen Fasermaterial FM und dem elektrischen Feld der Mikrowellen auswertbar ist, wobei diese Wechselwirkung bei einem zylindrischen Resonatorraum 5, bei dem die Zylinderachse ZA parallel zur Laufrichtung LR ist, in einem TM-Resonanzmodus besonders ausgeprägt ist. Der Index n gibt dabei an, wie viele Halbwellen das elektrische Feld in einer Richtung quer zur Zylinderachse ausbildet. Technisch bedingt beträgt n mindestens 1, da sich im entsprechenden Resonanzfall mindestens eine derartige Halbwelle ausbildet. Dabei hat sich jedoch gezeigt, dass sich das elektrische Feld im Messraum 5 günstiger, beispielsweise homogener ausbildet, wenn die Anzahl der Halbwellen erhöht wird, n also größer gewählt wird. Der Grund hierfür liegt darin, dass sich bei gegebener Geometrie des Mikrowellenresonators eine höhere Resonanzfrequenz ergibt.

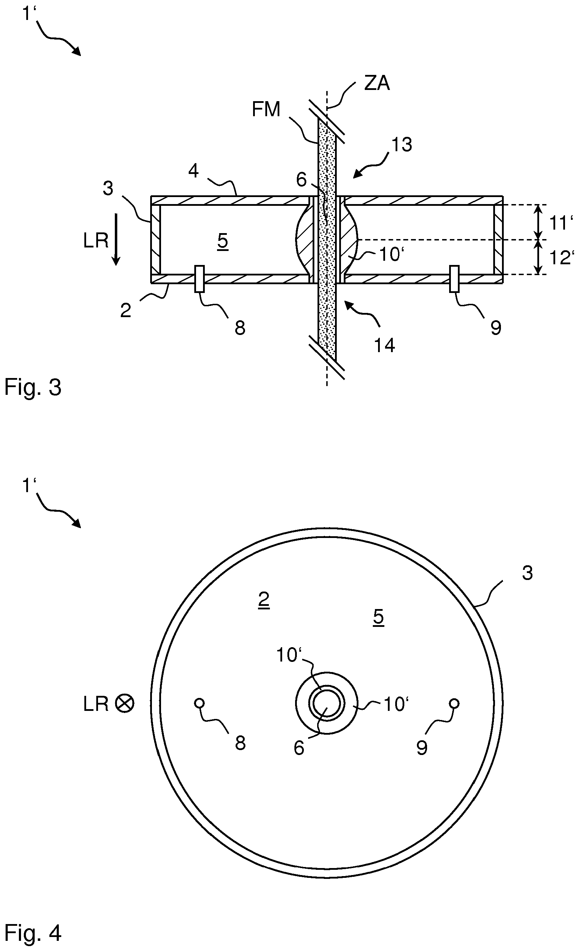

Figur 3 zeigt ein zweites Ausführungsbeispiel eines erfindungsgemäßen Mikrowellenresonators 1' in einer schematischen, geschnittenen Seitenansicht, welcher in Figur 4 in einer Aufsicht dargestellt ist. Der Mikrowellenresonator 1' entspricht im Wesentlichen dem Mikrowellenresonator 1 des ersten Ausführungsbeispiels. Im Folgenden werden lediglich die Unterschiede dazu erläutert.

Im zweiten Ausführungsbeispiel ist die Materialeinheit 10' als ein den Messraum 6 begrenzendes Begrenzungselement 7' zur Führung des Fasermaterials und/oder zur Abdeckung eines Hohlraumbereichs des Resonatorraums 5 ausgebildet. Das Begrenzungselement 7 der Figuren 1 und 2 entfällt hierbei. Durch die Zusammenfassung der feldhomogenisierenden Materialeinheit und einem Begrenzungselement kann der konstruktive Aufwand für den Aufbau des Mikrowellenresonators 1' verringert werden und gleichzeitig die gewünschte Homogenisierung des Felds erreicht werden.

Weiterhin ist im zweiten Ausführungsbeispiel vorgesehen, dass die Materialeinheit 10' eine zylindrische Öffnung, bevorzugt eine ellipsen- oder kreiszylindrische Öffnung, aufweist, die parallel, bevorzugt koaxial, zur Zylinderachse ZA des Resonatorraums 5 verläuft, wobei sich die Außenseite des eingangsseitigen Abschnitts 11' in Laufrichtung LR konvex gewölbt erweitert und/oder wobei sich die Außenseite des ausgangsseitigen Abschnitts 12' in Laufrichtung LR konvex gewölbt verjüngt. Mit dieser etwas aufwendiger herzustellenden Geometrie der Materialeinheit 10' kann, insbesondere bei einem zylindrischen Resonatorraum 5, bei dem die Laufrichtung LR des Fasermaterials FM parallel zu einer Zylinderachse ZA des Resonatorraums 5 verläuft, das elektrische Feld im Messraum 6 noch wirkungsvoller homogenisiert werden. Keramik, Glas und Verbundwerkstoffe mit eingebetteter Keramik sind zudem sehr abriebfest, so dass sie insbesondere für die Materialeinheit 10' geeignet sind, welche als Begrenzungselement 7' ausgebildet ist.

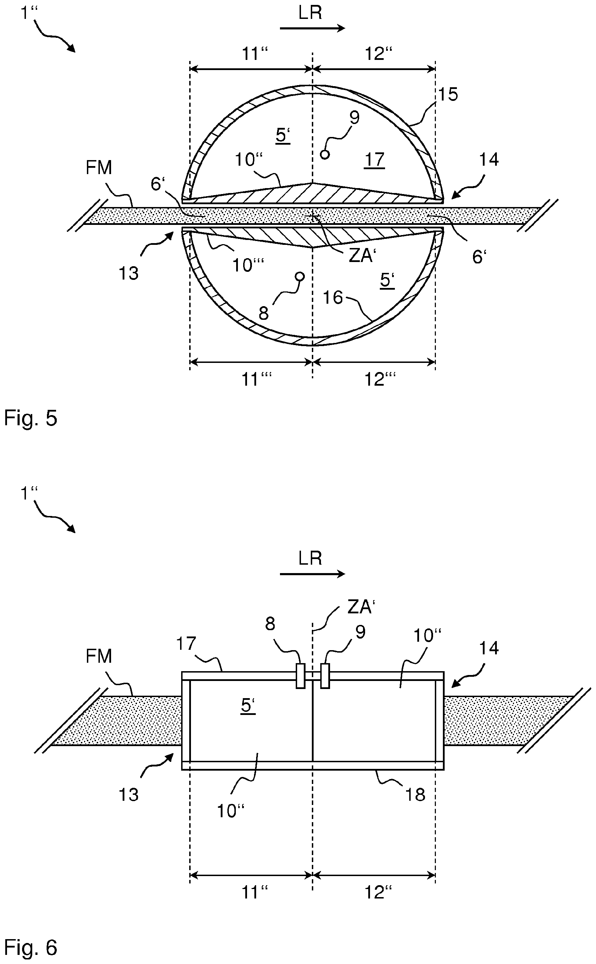

Figur 5 zeigt ein drittes Ausführungsbeispiel eines erfindungsgemäßen Mikrowellenresonators 1" in einer schematischen, geschnittenen Seitenansicht, welcher in Figur 6 in einer Aufsicht dargestellt ist. Der Mikrowellenresonator 1" entspricht im prinzipiellen Aufbau dem Mikrowellenresonator 1' des zweiten Ausführungsbeispiels. Im Folgenden werden lediglich die Unterschiede dazu erläutert.

Der Mikrowellenresonator 1" weist ein leitfähiges Gehäuse 15, 16, 17, 18 auf, welches durch eine obere Halbschale 15, eine untere Halbschale 16, eine Rückwand 17 und eine Vorderwand 18 gebildet ist.

Dabei ist vorgesehen, dass der Resonatorraum 5' zylindrisch, insbesondere ellipsenzylindrisch oder, wie gezeigt, kreiszylindrisch geformt ist, wobei die Laufrichtung LR des Fasermaterials FM' mit länglichem Querschnitt quer zu einer Zylinderachse ZA' des Resonatorraums 5' verläuft und wobei der Messraum 6' im Querschnitt als länglicher Schlitz 6' ausgebildet ist, wobei eine erste Materialeinheit 10" längs einer ersten Breitseite des Schlitzes 6' und eine zweite Materialeinheit 10‴ längs einer gegenüberliegenden zweiten Breitseite des Schlitzes 6' angeordnet ist, wobei bevorzugt die erste Materialeinheit 10" und die zweite Materialeinheit 10‴ bezüglich einer durch die Zylinderachse ZA' und parallel zur Laufrichtung LR verlaufenden Symmetrieebene spiegelsymmetrisch zueinander angeordnet sind. Zylindrische Resonatorräume 5', insbesondere ellipsenzylindrische oder kreiszylindrische Resonatorräume 5', bei denen die Laufrichtung LR des Fasermaterials FM quer zu einer Zylinderachse ZA' des Resonatorraums 5' verläuft und bei denen der Messraum 6' als länglicher Schlitz 6' ausgebildet ist, eignen sich insbesondere zur gemeinsamen Vermessung von mehreren nebeneinanderlaufenden strangförmigen Fasermaterialen oder, wie gezeigt, zur Vermessung eines Fasermaterials mit länglichem Querschnitt. In solchen Fällen kann das elektrische Feld im Messraum 6' durch eine erste Materialeinheit 10", welche längs einer ersten Breitseite des Schlitzes 6' angeordnet ist, und durch eine zweite Materialeinheit 10‴, welche längs einer gegenüberliegenden zweiten Breitseite des Schlitzes 6' angeordnet ist, also durch eine relativ einfache Anordnung von zwei Materialeinheiten 10", 10‴, beeinflusst, insbesondere homogenisiert, werden. Dabei kann eine besonders wirksame Beeinflussung des elektrischen Felds erreicht werden, wenn die erste Materialeinheit 10" und die zweite Materialeinheit 10‴ bezüglich einer durch die Zylinderachse ZA' und parallel zur Laufrichtung LR verlaufenden Symmetrieebene spiegelsymmetrisch zueinander angeordnet sind.

Zweckmäßigerweise ist vorgesehen, dass die erste Materialeinheit 10" und/oder die zweite Materialeinheit 10‴ eine parallel zur Zylinderachse ZA' verlaufende im Wesentlichen ebene Innenseite aufweist, wobei sich die Außenseite des jeweiligen eingangsseitigen Abschnitts 11", 11‴ in Laufrichtung LR keilförmig erweitert und/oder wobei sich die Außenseite des jeweiligen ausgangsseitigen Abschnitts 12", 12‴ in Laufrichtung LR keilförmig verjüngt. Mit dieser einfach herzustellenden Geometrie der Materialeinheiten 10", 10‴ kann, insbesondere bei einem zylindrischen Resonatorraum 5', bei dem die Laufrichtung LR des Fasermaterials FM quer zu einer Zylinderachse ZA' des Resonatorraums 5' verläuft, das elektrische Feld im Messraum 6' in besonders einfacher Weise günstig beeinflusst, beispielsweise homogenisiert, werden.

Vorteilhafterweise ist vorgesehen, dass der Mikrowellenresonator zum Betrieb in einem TE₀ₙₘ-Resonanzmodus ausgebildet ist, wobei n größer als 0, bevorzugt größer als 1, besonders bevorzugt größer als 2, ist. Unter einem TE-Resonanzmodus (Transversal Elektrischer Resonanzmodus) wird bei einem zylindrischen Resonatorraum allgemein ein Resonanzmodus verstanden, bei dem kein elektrisches Feld in Richtung der Zylinderachse auftritt. Dies ist insofern günstig, als bei der Vermessung von Fasermaterialien hauptsächlich die Wechselwirkung zwischen dem jeweiligen Fasermaterial und dem elektrischen Feld der Mikrowellen auswertbar ist, wobei diese Wechselwirkung bei einem zylindrischen Resonatorraum, bei dem die Zylinderachse quer zur Laufrichtung ist, in einem TE-Resonanzmodus besonders ausgeprägt ist. Der Index n gibt dabei an, wie viele Halbwellen das elektromagnetische Feld in einer Richtung quer zur Zylinderachse ausbildet. Technisch bedingt beträgt n mindestens 1, da sich im entsprechenden Resonanzfall mindesten eine derartige Halbwelle ausbildet. Dabei hat sich jedoch gezeigt, dass sich das elektrische Feld im Messraum homogener ausbildet, wenn die Anzahl der Halbwellen erhöht wird, n also größer gewählt wird. Wird n jedoch größer gewählt, so muss bei gegebenem Resonatorraum die Frequenz vergrößert werden. Dabei ergibt sich ein guter Kompromiss aus Größe und Homogenität, wenn n zwischen 2 und 4 gewählt wird. Der Index m ist die Anzahl der Halbwellen entlang der Zylinderachse und ist typischerweise gleich 1.

Es versteht sich von selbst, das bei dem Mikrowellenresonator 1" analog zum Mikrowellenresonator 1 ein oder mehrere Begrenzungselemente 7 vorgesehen sein könnten.

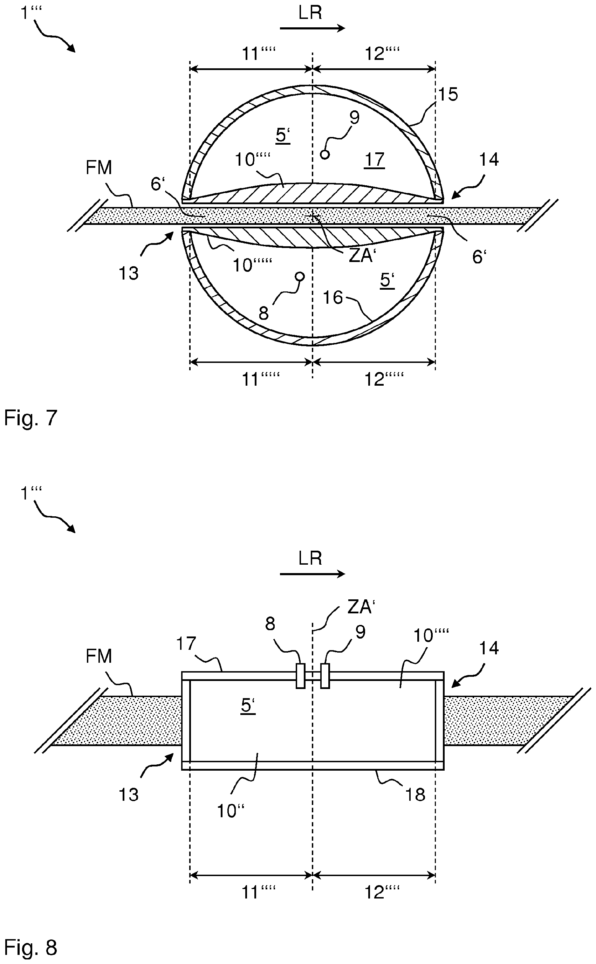

Figur 7 zeigt ein viertes Ausführungsbeispiel eines erfindungsgemäßen Mikrowellenresonators 1‴ in einer schematischen, geschnittenen Seitenansicht, welcher in Figur 8 in einer Aufsicht dargestellt ist. Der Mikrowellenresonator 1‴ entspricht im prinzipiellen Aufbau dem Mikrowellenresonator 1" des dritten Ausführungsbeispiels. Im Folgenden werden lediglich die Unterschiede dazu erläutert.

Im vierten Ausführungsbeispiel ist vorgesehen, dass die erste Materialeinheit 10"" und/oder die zweite Materialeinheit 10""' eine parallel zur Zylinderachse ZA' verlaufende im Wesentlichen ebene Innenseite aufweist, wobei sich die Außenseite des jeweiligen eingangsseitigen Abschnitts 11"", 11""' in Laufrichtung LR konvex gewölbt erweitert und/oder wobei sich die Außenseite des jeweiligen ausgangsseitigen Abschnitts 12"", 12'"" in Laufrichtung LR konvex gewölbt verjüngt. Mit dieser etwas aufwendiger herzustellenden Geometrie der Materialeinheiten 10"", 10'"" kann insbesondere bei einem zylindrischen Resonatorraum 5', bei dem die Laufrichtung LR des Fasermaterials FM quer zu einer Zylinderachse ZA' des Resonatorraums 5' verläuft, das elektrische Feld im Messraum 6' noch wirkungsvoller beeinflusst, beispielsweise homogenisiert, werden.

## Patentansprüche

1. Mikrowellenresonator für eine Messeinrichtung zur Messung der Dicke und/oder der Feuchtigkeit von kontinuierlich durch einen in einem Resonatorraum (5, 5') des Mikrowellenresonators (1, 1', 1", 1‴) angeordneten Messraum (6, 6') gefördertem strangförmigem Fasermaterial (FM) an einer Textilmaschine, insbesondere an einer Karde, an einer Strecke oder an einer Kämmmaschine, wobei in dem Resonatorraum (5, 5') längs des Messraums (6, 6') wenigstens eine elektrisch nicht leitende Materialeinheit (10, 10', 10", 10‴, 10"", 10'"") vorgesehen ist,
welche einen eingangsseitigen Abschnitt (11, 11', 11", 11‴, 11"", 11'"") mit einer in einer vorgesehenen Laufrichtung (LR) des Fasermaterials (FM) veränderlichen Querschnittsfläche und/oder einen ausgangsseitigen Abschnitt (12, 12', 12", 12‴, 12"", 12'"") mit einer in der vorgesehenen Laufrichtung (LR) des Fasermaterials (FM) veränderlichen Querschnittsfläche aufweist,
wobei die Querschnittsfläche des eingangsseitigen Abschnitts (11, 11', 11", 11‴, 11"", 11'"") der Materialeinheit (10, 10', 10", 10‴, 10"", 10'"") in der Laufrichtung (LR) des Fasermaterials (FM) gesehen zunimmt und/oder die Querschnittsfläche des ausgangsseitigen Abschnitts (12, 12', 12", 12‴, 12"", 12'"") der Materialeinheit (10, 10', 10", 10‴, 10"", 10'"") in der Laufrichtung (LR) des Fasermaterials (FM) gesehen abnimmt,
**dadurch gekennzeichnet, dass**
die Materialeinheit (10', 10", 10‴, 10"", 10'"") zur Abdeckung eines Hohlraumbereichs des Resonatorraums (5, 5') ausgebildet ist.

2. Mikrowellenresonator nach dem vorherigen Anspruch, **dadurch gekennzeichnet, dass** die Querschnittsfläche des eingangsseitigen Abschnitts (11, 11', 11", 11‴, 11"", 11'"") der Materialeinheit (10, 10', 10", 10‴, 10"", 10""') kontinuierlich zunimmt und/oder dass die Querschnittsfläche des ausgangsseitigen Abschnitts (12, 12', 12", 12‴, 12"", 12'"") der Materialeinheit (10, 10', 10", 10‴, 10"", 10'"") kontinuierlich abnimmt.

3. Mikrowellenresonator nach einem der vorherigen Ansprüche,
**dadurch gekennzeichnet, dass** der eingangsseitige Abschnitt (11, 11', 11", 11‴, 11"", 11'"") der Materialeinheit (10, 10', 10", 10‴, 10"", 10'"") und der ausgangsseitige Abschnitt (12, 12', 12", 12‴, 12"", 12'"") der Materialeinheit (10, 10', 10", 10‴, 10"", 10'"") aneinander angrenzen.

4. Mikrowellenresonator nach einem der vorherigen Ansprüche,
**dadurch gekennzeichnet, dass** sich der eingangsseitige Abschnitt (11, 11', 11", 11‴, 11"", 11'"") von einem Eintrittsbereich (13) des Fasermaterials (FM) in den Resonatorraum (5, 5') bis zu einem mittleren Bereich des Resonatorraums (5, 5') erstreckt und/oder dass sich der ausgangsseitige Abschnitt (12, 12', 12", 12‴, 12"", 12'"") von dem mittleren Bereich des Resonatorraums (5, 5') bis zu einem Austrittsbereich (14) des Fasermaterials (FM) aus dem Resonatorraum (5, 5') erstreckt.

5. Mikrowellenresonator nach einem der vorherigen Ansprüche,
**dadurch gekennzeichnet, dass** der eingangsseitige Abschnitt (11, 11', 11", 11‴, 11"", 11'"") und der ausgangsseitige Abschnitt (12, 12', 12", 12‴, 12"", 12'"") spiegelsymmetrisch zueinander ausgebildet sind.

6. Mikrowellenresonator nach einem der vorherigen Ansprüche,
**dadurch gekennzeichnet, dass** die Materialeinheit (10) an der dem Messraum (6, 6') abgewandten Seite eines den Messraum (6, 6') begrenzenden Begrenzungselements (7) zur Führung des Fasermaterials (FM) und/oder zur Abdeckung eines Hohlraumbereichs des Resonatorraums (5) angeordnet ist.

7. Mikrowellenresonator nach einem der Ansprüche 1 bis 6, **dadurch gekennzeichnet, dass** die Materialeinheit (10', 10", 10‴, 10"", 10'"") als ein den Messraum (6, 6') begrenzendes Begrenzungselement (7', 7", 7‴, 7"", 7""') zur Führung des Fasermaterials (FM) ausgebildet ist, und/oder dass die Materialeinheit (10, 10', 10", 10‴, 10"", 10'"") aus einer Keramik, insbesondere aus Aluminiumoxid, aus einem Kunststoff, insbesondere aus Polyethylen, aus einem Glas, insbesondere aus Quarzglas, oder aus einem Verbundwerkstoff mit eingebetteter Keramik hergestellt ist.

8. Mikrowellenresonator nach einem der vorherigen Ansprüche,
**dadurch gekennzeichnet, dass** der Resonatorraum (5) zylindrisch, insbesondere ellipsenzylindrisch oder kreiszylindrisch geformt ist, wobei die Laufrichtung (LR) des Fasermaterials (FM) parallel zu einer Zylinderachse (ZA) des Resonatorraums (5) verläuft und wobei bevorzugt die Materialeinheit (10, 10') rotationssymmetrisch ausgebildet und koaxial zur Zylinderachse (ZA) angeordnet ist, und/oder dass die Materialeinheit (10) eine zylindrische Öffnung, bevorzugt eine ellipsen- oder kreiszylindrische Öffnung aufweist, die parallel, bevorzugt koaxial, zur Zylinderachse (ZA) des Resonatorraums (5) verläuft, wobei sich die Außenseite des eingangsseitigen Abschnitts (11) in Laufrichtung (LR) konisch erweitert und/oder wobei sich die Außenseite des ausgangsseitigen Abschnitts (12) in Laufrichtung (LR) konisch verjüngt.

9. Mikrowellenresonator nach Anspruch 8, **dadurch gekennzeichnet, dass** die Materialeinheit (10') eine zylindrische Öffnung, bevorzugt eine ellipsen- oder kreiszylindrische Öffnung, aufweist, die parallel, bevorzugt koaxial, zur Zylinderachse (ZA) des Resonatorraums (5) verläuft, wobei sich die Außenseite des eingangsseitigen Abschnitts (11') in Laufrichtung (LR) konvex gewölbt erweitert und/oder wobei sich die Außenseite des ausgangsseitigen Abschnitts (12') in Laufrichtung konvex gewölbt verjüngt.

10. Mikrowellenresonator nach einem der Ansprüche 8 oder 9, **dadurch gekennzeichnet, dass** er zum Betrieb in einem TM₀ₙ₀-Resonanzmodus ausgebildet ist, wobei n eine natürliche Zahl, bevorzugt größer als 1, und besonders bevorzugt größer als 2, ist.

11. Mikrowellenresonator nach einem der Ansprüche 1 bis 8, **dadurch gekennzeichnet, dass** der Resonatorraum (5') zylindrisch, insbesondere ellipsenzylindrisch oder kreiszylindrisch geformt ist, wobei die Laufrichtung (LR) des Fasermaterials (FM) quer zu einer Zylinderachse (ZA') des Resonatorraums (5') verläuft und wobei der Messraum (6') im Querschnitt als länglicher Schlitz (6') ausgebildet ist, wobei eine erste Materialeinheit (10", 10"") längs einer ersten Breitseite des Schlitzes (6') und eine zweite Materialeinheit (10‴, 10'"") längs einer gegenüberliegenden zweiten Breitseite des Schlitzes (6') angeordnet ist, wobei bevorzugt die erste Materialeinheit (10", 10"") und die zweite Materialeinheit (10‴, 10'"") bezüglich einer durch die Zylinderachse (ZA') und parallel zur Laufrichtung (LR) verlaufenden Symmetrieebene spiegelsymmetrisch zueinander angeordnet sind, wobei die erste Materialeinheit (10") und/oder die zweite Materialeinheit (10‴) vorzugsweise eine parallel zur Zylinderachse (ZA') verlaufende im Wesentlichen ebene Innenseite aufweist, wobei sich die Außenseite des jeweiligen eingangsseitigen Abschnitts (11", 11‴) in Laufrichtung (LR) vorzugsweise keilförmig erweitert und/oder wobei sich die Außenseite des jeweiligen ausgangsseitigen Abschnitts (12", 12‴) in Laufrichtung (LR) vorzugsweise keilförmig verjüngt.

12. Mikrowellenresonator nach Anspruch 11, **dadurch gekennzeichnet, dass** die erste Materialeinheit (10"") und/oder die zweite Materialeinheit (10""') eine parallel zur Zylinderachse verlaufende im Wesentlichen ebene Innenseite aufweist, wobei sich die Außenseite des jeweiligen eingangsseitigen Abschnitts (11"", 11""') in Laufrichtung (LR) konvex gewölbt erweitert und/oder wobei sich die Außenseite des jeweiligen ausgangsseitigen Abschnitts (12"", 12'"") in Laufrichtung (LR) konvex gewölbt verjüngt.

13. Mikrowellenresonator nach Anspruch 11 oder 12, **dadurch gekennzeichnet, dass** er zum Betrieb in einem TE₀ₙₘ-Resonanzmodus ausgebildet ist, wobei n größer als 0, bevorzugt größer als 1 und besonders bevorzugt größer als 2 ist.

14. Textilmaschine, insbesondere Karde, Strecke oder Kämmmaschine, **gekennzeichnet durch** mindestens einen Mikrowellenresonator (1, 1', 1", 1‴) nach einem der vorhergehenden Ansprüche.

## Claims

1. A microwave resonator for a measuring device for measuring the thickness and/or moisture of strand-shaped fiber material (FM) continuously conveyed through a measurement chamber (6, 6') disposed in a resonator chamber (5, 5') of the microwave resonator (1, 1', 1", 1‴) on a textile machine, particularly on a carding machine, on a drawing frame, or on a combing machine, wherein at least one electrically nonconductive material unit (10, 10', 10", 10"', 10"", 10""') is provided in the resonator chamber (5, 5') along the measurement chamber (6, 6'), said material unit comprising an inlet-side section (11, 11', 11", 11‴, 11"", 11""') having a cross-sectional area that is variable in an intended travel direction (LR) of the fiber material (FM) and/or an outlet-side section (12, 12', 12", 12‴, 12"", 12""') having a cross-sectional area that is variable in the intended travel direction (LR) of the fiber material (FM),
wherein the cross-sectional area of the inlet-side section (11, 11', 11", 11‴, 11"", 11""') of the material unit (10, 10', 10", 10'", 10"", 10""') increases in the travel direction (LR) of the fiber material (FM) and/or the cross-sectional area of the outlet-side section (12, 12', 12", 12‴, 12"", 12""') of the material unit (10, 10', 10", 10‴, 10"", 10""') decreases in the travel direction (LR) of the fiber material (FM)
**characterized in that**
the material unit (10', 10", 10‴, 10""' 10""') is implemented for covering a hollow space region of the resonator chamber (5, 5').

2. The microwave resonator according to the preceding claim,
**characterized in that** the cross-sectional area of the inlet-side section (11, 11', 11", 11‴, 11"", 11""') of the material unit (10, 10', 10", 10‴, 10"", 10""') increases continuously and/or that the cross-sectional area of the outlet-side section (12, 12', 12", 12‴, 12"", 12""') of the material unit (10, 10', 10", 10‴, 10"", 10""') decreases continuously.

3. The microwave resonator according to any one of the preceding claims, **characterized in that** the inlet-side section (11, 11', 11", 11‴, 11"", 11""') of the material unit (10, 10', 10", 10‴, 10"", 10""') and the outlet-side section (12, 12', 12", 12‴, 12"", 12""') of the material unit (10, 10', 10", 10‴, 10"", 10""') border each other.

4. The microwave resonator according to any one of the preceding claims, **characterized in that** the inlet-side section (11, 11', 11", 11‴, 11"", 11""') extends from an entry region (13) of the fiber material (FM) into the resonator chamber (5, 5') to a middle region of the resonator chamber (5, 5') and/or that the outlet-side section (12, 12', 12", 12‴, 12"", 12""') extends from the middle region of the resonator chamber (5, 5') to an exit region (14) of the fiber material (FM) out of the resonator chamber (5, 5').

5. The microwave resonator according to any one of the preceding claims, **characterized in that** the inlet-side section (11, 11', 11", 11‴, 11"", 11""') and the outlet-side section (12, 12', 12", 12‴, 12"", 12""') of the material unit (10, 10', 10", 10‴, 10"", 10""') are formed mirror symmetrically relative to each other.

6. The microwave resonator according to any one of the preceding claims, **characterized in that** the material unit (10) is disposed on the side facing away from the measurement chamber (6, 6') of a bounding element (7) bounding the measurement chamber (6, 6') for guiding the fiber material (FM) and/or for covering a hollow space region of the resonator chamber (5).

7. The microwave resonator according to any one of the claims 1 through 6, **characterized in that** the material unit (10', 10", 10‴, 10"", 10""') is implemented as a bounding element (7', 7", 7‴, 7"", 7""'), for guiding the fiber material (FM), that is bounding the measurement chamber (6, 6') and/or that the material unit (10, 10', 10", 10‴, 10"", 10""') is made of a ceramic, particularly of aluminum oxide, of a plastic, particularly of polyethylene, of a glass, particularly of quartz glass, or of a composite material having embedded ceramics.

8. The microwave resonator according to any one of the preceding claims, **characterized in that** the resonator chamber (5) is cylindrical in shape, particularly elliptical cylindrical or circular cylindrical, wherein the travel direction (LR) of the fiber material (FM) runs parallel to a cylinder axis (ZA) of the resonator chamber (5) and wherein the material unit (10, 10') is implemented rotationally symmetrically and coaxial to the cylinder axis (ZA) and/or that the material unit (10) comprises a cylindrical opening, preferably an elliptical cylindrical or circular cylindrical opening, running parallel, preferably coaxial, to the cylinder axis (ZA) of the resonator chamber (5), wherein the outer side of the inlet-side section (11) widens conically in the travel direction (LR) and/or wherein the outer side of the outlet-side section (12) tapers conically in the travel direction (LR).

9. The microwave resonator according to claim 8, **characterized in that** the material unit (10') comprises a cylindrical opening, preferably an elliptical cylindrical or circular cylindrical opening, running parallel, preferably coaxial, to the cylinder axis (ZA) of the resonator chamber (5), wherein the outer side of the inlet-side section (11') widens in a convex curve in the travel direction (LR) and/or wherein the outer side of the outlet-side section (12') tapers in a convex curve in the travel direction.

10. The microwave resonator according to any one of the claims 8 or 9, **characterized in that** said microwave resonator is implemented for operating in a TM₀ₙ₀ resonance mode, wherein n is a natural number, preferably greater than 1, and particularly preferably greater than 2.

11. The microwave resonator according to any one of the claims 1 through 8, **characterized in that** the resonator chamber (5') is cylindrical in shape, particularly elliptical cylindrical or circular cylindrical, wherein the travel direction (LR) of the fiber material (FM) runs transverse to a cylinder axis (ZA') of the resonator chamber (5') and wherein the measurement chamber (6') is implemented as an elongated slit (6') in cross section, wherein a first material unit (10", 10"") is disposed along a first wide side of the slit (6') and a second material unit (10"', 10""') is disposed along an opposite second wide side of the slit (6'), wherein the first material unit (10", 10"") and the second material unit (10‴, 10""') are preferably disposed mirror symmetrically relative to each other with respect to a plane of symmetry running through the cylinder axis (ZA') and parallel to the travel direction (LR) wherein the first material unit (10") and/or the second material unit (10‴) preferably comprise a substantially flat inner side running parallel to the cylinder axis (ZA'), wherein the outer side of the respective inlet-side section (11", 11‴) widens in a wedge shape in the travel direction (LR) and/or wherein the outer side of the respective outlet-side section (12", 12‴) tapers in a wedge shape in the travel direction (LR).

12. The microwave resonator according to claim 11, **characterized in that** the first material unit (10"") and/or the second material unit (10""') comprises a substantially flat inner side running parallel to the cylinder axis, wherein the outer side of the respective inlet-side section (11"", 11""') widens in a convex curve in the travel direction (LR) and/or wherein the outer side of the respective outlet-side section (12"", 12""') tapers in a convex curve in the travel direction (LR).

13. The microwave resonator according to any one of the claims 11 or 12, **characterized in that** said microwave resonator is implemented for operating in a TE₀ₙₘ resonance mode, wherein n is greater than 0, preferably greater than 1, and particularly preferably greater than 2.

14. A textile machine, particularly a carding machine, drawing frame, or combing machine, **characterized by** at least one microwave resonator (1, 1', 1", 1‴) according to any one of the preceding claims.

## Revendications

1. Résonateur à hyperfréquence pour un dispositif de mesure destiné à mesurer l'épaisseur et/ou l'humidité d'une matière fibreuse en forme d'écheveau (FM) transporté en continu à travers un espace de mesure (6, 6') disposé dans un espace de résonateur (5, 5') du résonateur à hyperfréquence (1, 1', 1", 1‴) sur une machine textile, en particulier sur une cardeuse, sur une étireuse ou sur une peigneuse, dans lequel au moins une unité de matériau (10, 10', 10", 10‴, 10"", 10""') non conducteur électriquement est prévue dans l'espace de résonateur (5, 5') le long de l'espace de mesure (6, 6'), laquelle présente une section côté entrée (11, 11', 11", 11‴, 11"", 11""') avec une surface de section transversale variable dans une direction de défilement prévue (LR) de la matière fibreuse (FM) et/ou une section côté sortie (12, 12', 12", 12‴, 12"", 12""') avec une surface de section transversale variable dans la direction de défilement prévue (LR) de la matière fibreuse (FM),
dans lequel la surface de section transversale de la section côté entrée (11, 11', 11", 11‴, 11"", 11'"") de l'unité de matériau (10, 10', 10", 10‴, 10"", 10""') augmente dans la direction de défilement (LR) de la matière fibreuse (FM) et/ou la surface de section transversale de la section côté sortie (12, 12', 12", 12‴, 12"", 12""') de l'unité de matériau (10, 10', 10", 10‴, 10"", 10""') diminue dans la direction de défilement (LR) de la matière fibreuse (FM),
**caractérisé en ce que**
l'unité de matériau (10', 10", 10‴, 10"", 10'"") est conçue pour recouvrir une zone d'espace creux de l'espace de résonateur (5, 5').

2. Résonateur à hyperfréquence selon la revendication précédente, **caractérisé en ce que** la surface de section transversale de la section côté entrée (11, 11', 11", 11‴, 11"", 11'"") de l'unité de matériau (10, 10', 10", 10‴, 10"", 10""') augmente continuellement et/ou que la surface de section transversale de la section côté sortie (12, 12', 12", 12‴, 12"", 12""') de l'unité de matériau (10, 10', 10", 10‴, 10"", 10'"") diminue continuellement.

3. Résonateur à hyperfréquence selon l'une quelconque des revendications précédentes, **caractérisé en ce que** la section côté entrée (11, 11', 11", 11‴, 11"", 11'"") de l'unité de matériau (10, 10', 10", 10‴, 10"", 10""') et la section côté sortie (12, 12', 12", 12‴, 12"", 12""') de l'unité de matériau (10, 10', 10", 10‴, 10"", 10'"") sont attenantes.

4. Résonateur à hyperfréquence selon l'une quelconque des revendications précédentes, **caractérisé en ce que** la section côté entrée (11, 11', 11", 11‴, 11"", 11'"") s'étend depuis une zone d'entrée (13) de la matière fibreuse (FM) dans l'espace de résonateur (5, 5') jusqu'à une zone centrale de l'espace de résonateur (5, 5') et/ou **en ce que** la section côté sortie (12, 12', 12", 12‴, 12"", 12""') s'étend de la zone centrale de l'espace de résonateur (5, 5') jusqu'à une zone de sortie (14) de la matière fibreuse (FM) de l'espace de résonateur (5, 5').

5. Résonateur à hyperfréquence selon l'une quelconque des revendications précédentes, **caractérisé en ce que** la section côté entrée (11, 11', 11", 11‴, 11"", 11'"") et la section côté sortie (12, 12', 12", 12‴, 12"", 12""') sont réalisées avec une symétrie miroir l'une par rapport à l'autre.

6. Résonateur à hyperfréquence selon l'une quelconque des revendications précédentes, **caractérisé en ce que** l'unité de matériau (10) est disposée sur le côté détourné de l'espace de mesure (6, 6') d'un élément de délimitation (7) délimitant l'espace de mesure (6, 6') pour guider la matière fibreuse (FM) et/ou pour recouvrir une zone d'espace creux de l'espace de résonateur (5).

7. Résonateur à hyperfréquence selon l'une quelconque des revendications 1 à 6, **caractérisé en ce que** l'unité de matériau (10', 10", 10‴, 10"", 10""') est conçue comme un élément de délimitation (7', 7", 7‴, 7"", 7""') délimitant l'espace de mesure (6, 6') pour guider la matière fibreuse (FM), et/ou **en ce que** l'unité de matériau (10, 10', 10", 10‴, 10"", 10""') est réalisé en une céramique, en particulier en oxyde d'aluminium, en une matière plastique, en particulier en polyéthylène, en un verre, en particulier en verre de quartz, ou en un matériau composite avec céramique incorporée.

8. Résonateur à hyperfréquence selon l'une quelconque des revendications précédentes, **caractérisé en ce que** l'espace de résonateur (5) est de forme cylindrique, en particulier cylindrique elliptique ou cylindrique circulaire, dans lequel la direction de défilement (LR) de la matière fibreuse (FM) s'étend parallèlement à un axe de cylindre (ZA) de l'espace de résonateur (5) et dans lequel, de préférence, l'unité de matériau (10, 10') est réalisée de manière rotationnellement symétrique et disposée coaxialement à l'axe de cylindre (ZA), et/ou **en ce que** l'unité de matériau (10) présente une ouverture cylindrique, de préférence une ouverture cylindrique elliptique ou cylindrique circulaire, qui s'étend parallèlement, de préférence coaxialement, à l'axe de cylindre (ZA) de l'espace de résonateur (5), dans lequel le côté extérieur de la section côté entrée (11) s'élargit de manière conique dans la direction de défilement (LR) et/ou le côté extérieur de la section côté sortie (12) se rétrécit de manière conique dans la direction de défilement (LR).

9. Résonateur à hyperfréquence selon la revendication 8, **caractérisé en ce que** l'unité de matériau (10') présente une ouverture cylindrique, de préférence une ouverture cylindrique elliptique ou cylindrique circulaire, qui s'étend parallèlement, de préférence coaxialement, à l'axe de cylindre (ZA) de l'espace de résonateur (5), dans lequel le côté extérieur de la section côté entrée (11') s'élargit de manière bombée convexe dans la direction de défilement (LR) et/ou dans lequel le côté extérieur de la section côté sortie (12') se rétrécit de manière bombée convexe dans la direction de défilement.

10. Résonateur à hyperfréquence selon l'une quelconque des revendications 8 ou 9, **caractérisé en ce qu'**il est conçu pour fonctionner dans un mode de résonance TM₀ₙ₀, sachant que n est un nombre naturel, de préférence supérieur à 1, et de manière particulièrement préférée supérieur à 2.

11. Résonateur à hyperfréquence selon l'une quelconque des revendications 1 à 8, **caractérisé en ce que** l'espace de résonateur (5') est de forme cylindrique, en particulier cylindrique elliptique ou cylindrique circulaire, dans lequel la direction de défilement (LR) de la matière fibreuse (FM) s'étendant perpendiculairement à un axe de cylindre (ZA') de l'espace de résonateur (5') et dans lequel l'espace de mesure (6') est conçue en section transversale comme une fente allongée (6'), dans lequel une première unité de matériau (10", 10"") est disposée le long d'un premier grand côté de la fente (6') et une deuxième unité de matériau (10‴, 10""') est disposée le long d'un deuxième grand côté opposé de la fente (6'), dans lequel, de préférence, la première unité de matériau (10", 10"") et la deuxième unité de matériau (10‴, 10""') sont disposées de manière symétrique l'une par rapport à l'autre par rapport à un plan de symétrie s'étendant par l'axe de cylindre (ZA') et parallèle à la direction de défilement (LR), dans lequel la première unité de matériau (10") et/ou la deuxième unité de matériau (10‴) présente(n) de préférence une face intérieure sensiblement plane s'étendant parallèlement à l'axe de cylindre (ZA'), dans lequel la face extérieure de la section côté entrée respective (11", 11‴) s'élargit de préférence de manière cunéiforme dans la direction de défilement (LR) et/ou dans lequel la face extérieure de la section côté sortie respective (12", 12‴) se rétrécit de préférence de manière cunéiforme dans la direction de défilement (LR).

12. Résonateur à hyperfréquence selon la revendication 11, **caractérisé en ce que** la première unité de matériau (10"") et/ou la deuxième unité de matériau (10""') présente(n) une face intérieure essentiellement plane s'étendant parallèlement à l'axe de cylindre, dans lequel la face extérieure de la section côté entrée respective (11"", 11""') s'élargit de manière bombée convexe dans la direction de défilement (LR) et/ou la face extérieure de la section côté sortie respective (12"", 12""') se rétrécit de manière bombée convexe dans la direction de défilement (LR).

13. Résonateur à hyperfréquence selon la revendication 11 ou 12, caractérisé en ce qu'il est conçu pour fonctionner dans un mode de résonance TE₀ₙₘ, sachant que n est supérieur à 0, de préférence supérieur à 1 et de manière particulièrement préférée supérieur à 2.

14. Machine textile, en particulier cardeuse, étireuse ou peigneuse, **caractérisée par** au moins un résonateur à hyperfréquence (1, 1', 1", 1‴) selon l'une quelconque des revendications précédentes.
